# EUROPEAN PATENT APPLICATION

(11) **EP 2 428 219 A1**
(43) Date of publication of application: **14.03.2012**
(21) Application number: 10175866.2
(22) Date of filing: 08.09.2010
(51) Int. Cl.: A61K 39/00

(54) **Methods for the treatment or prevention of autoimmune diseases or autoinflammatory diseases**

(71) Applicant: Stichting Katholieke Universiteit, 6500 HB Nijmegen (NL)
(72) Inventor: Radstake, Timothy Ruben Dirk Jan, 6500 HB, Nijmegen (NL)
(74) Representative: Habets, Winand

(57) **Abstract**

The invention is in the field of molecular immunology, more in particular in the field of the prevention or treatment of autoimmune diseases and autoinflammatory diseases, more in particular systemic sclerosis or scleroderma. The invention is based on the observation that such patients have an elevated plasma level of CXCL4. This was found to contribute to the pathogenesis of autoimmune and/or autoinflammatory diseases such as rheumatoid arthritis, psoriatic arthritis, lupus erythematosus, systemic sclerosis, multiple sclerosis, etc When CXCL4 was neutralized in in vitro experiments, the fibrotic effects could be neutralized. This led us to conclude that autoimmune and/or autoinflammatory diseases such as rheumatoid arthritis, psoriatic arthritis, lupus erythematosus, systemic sclerosis, multiple sclerosis, etc may be cured by reducing the plasma level of CXCL4. The invention therefore relates to a method for treatment or prevention of AID such as rheumatoid arthritis, psoriatic arthritis, lupus erythematosus, systemic sclerosis, multiple sclerosis, etc wherein the plasma level of CXCL4 is reduced.

## Description

### Field of the invention

The invention is in the field of molecular immunology, more in particular in the field of the prevention or treatment of autoimmune or autoinflammatory diseases.

### Background of the invention

Autoimmune and/or autoinflammatory diseases are frequently occurring and have several different modes of onset. However, one final common pathway involves the Toll-like receptor (TLR) pathway. TLR are well-known for their capacity to recognize micro-organisms such as bacteria and viruses (Pattern associated molecular pattern (PAMPs)) upon which they elicit a very potent downstream activation signal that culminates in cell activation, secretion of inflammatory mediators and finally in a chronic inflammatory response damaging its host. The last few years it became clear that TLR also recognize a variety of molecules that are released upon cell damage; the so-called damage associated molecular patterns (DAMPs). Thus, the TLR system is pivotal in the control and initiation of our immune system. Although the precise underlying pathways that lead to the breakthrough of tolerance in autoimmunity or, the chronic never abating inflammatory response in autoinflammatory conditions, are paradigmatic, it is clear that TLR signaling plays an central contributing role in many of these conditions. Moreover, in various diseases such as RA and psoriatic arthritis, the TLR system was demonstrated to react more potently to a variety of stimuli compared to that in healthy controls. Therefore, a mechanism that could restore a normal TLR response could possibly function as a novel therapeutic target for a variety of medical conditions covering the wide spectrum of medicine

### Summary of the invention

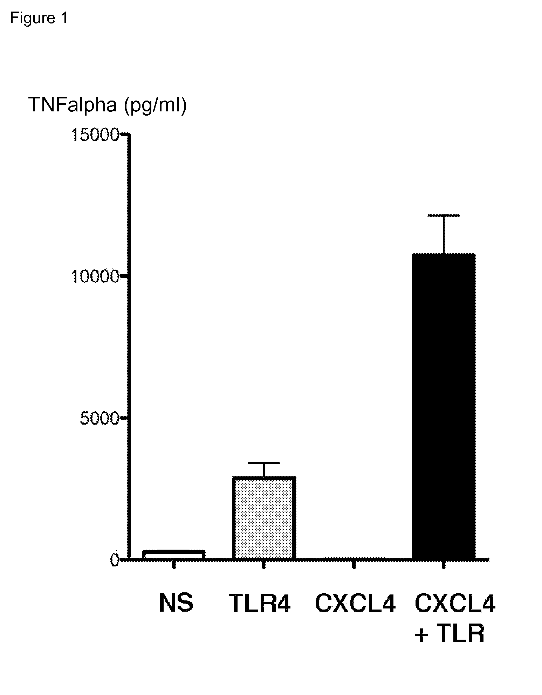

The invention is based on the observation that patients with systemic sclerosis (SSC) had a potentiated TLR response in vitro. After blocking the chemokine CXCL4, this potentiated response was normalized and behaved similar as observed as in healthy individuals. Similarly, dendritic cells (DCs) co-cultured with CXCL4 showed a markedly enhance response to TLR response whereas when unstimulated, CXCL4 stimulated DCs displayed a low inflammatory level as observed by their low production of inflammatory mediators (figure 1). Further research revealed that CXCL4 probably puts forward its function by the downregulation of inhibitory TLR adaptor molecules such as A20, SOCS3 and SARM. CXCL4 thus plays a central role in the regulation of the magnitude of TLR responses.

Our findings show that when a cell enters an inflammatory site where CXCL4 is present in large quantities, this will further enhance the inflammatory loop leading to a self-perpetuating loop of inflammation. We found that removing or inactivating CXCL4 from the circulation has an anti-inflammatory effect.

The invention therefore relates to a compound capable of binding to CXCL4 for use in the treatment or prevention of fibrosis in patients with autoimmune diseases (AID).

The invention also relates to an vitro method for reducing the level of CXCL4 and/or the number of pDCs in a sample obtained from a patient with scleroderma, comprising the steps of
a. Providing said sample obtained from a patient with scleroderma
b. Contacting the sample with a compound capable of binding to CXCL4
c. Allowing the compound to bind to CXCL4 in order to form a complex.

### Detailed description of the invention

We have found that CXCL4 is able to change the behavior of macrofages and dendritic cells. It has previously been show that CXCL4 alters macrophage function (Gleissner et al., Circulation Research. 2010; 106: 203-211 and J. Immunol. 2010 184: 4810 -4818).

However, in these studies, CXCL4 always led to an anti-inflammatory response. Here we confirm these observations showing that the presence of CXCL4 may drive an anti-inflammatory response, but only in the absence of any other stimulant. However, and in sharp contrast with the literature, when CXCL4-stimulated DC and/or macrophages are subsequently stimulated with TLR ligands, then these cells display a markedly increased capacity to produce inflammatory mediators (Figure 1). This effect was present when DCs / macrophages were stimulated with CXCL4 from the beginning, immediately after isolation of their precursor cells but also after adding CXCL4 after 3 days of culture.

The latter shows that when a cell enters an inflammatory site where CXCL4 is present in large quantities, this may further enhance the inflammatory loop leading to a self-perpetuating loop of inflammation. This observation was true for all TLR subtypes tested (TLR2, TLR3, TLR4, TLR7, TLR8 and TLR9) and was applicable on all inflammatory mediators tested (IL-12, IL-6, TNFa).

Hence, autoimmune diseases and/or autoinflammatory diseases may be treated by neutralizing the function of CXCL4, for instance in by depleting CXCL4 from the circulation of these patients.

Patients with AID such as rheumatoid arthritis, psoriatic arthritis, lupus erythematosus, systemic sclerosis, multiple sclerosis, etc may therefore benefit from methods that remove CXCL4, for instance from the blood circulation. That may be accomplished in a number of ways, which are known in the art per se.

Patients with autoimmune and/or autoinflammatory diseases may benefit from a treatment wherein the effects of the CXCL4 produced in excess are neutralized or counteracted, for instance by removing CXCL4 from circulation. In the alternative, CXCL4 may be prevented from binding to its receptor. The invention therefore relates to a compound capable of binding to CXCL4 for use in the treatment or prevention of fibrosis in patients with autoimmune and/or autoinflammatory diseases such as rheumatoid arthritis, psoriatic arthritis, lupus erythematosus, systemic sclerosis, multiple sclerosis, etc.

Compounds capable of binding to CXCL4 may be compounds capable of binding to CXCL4 under physiological conditions.

Compounds capable of binding to CXCL4 per se are known in the art. For instance, fragments of the receptor or fragments of CXCL4 will be suitable compounds. In a preferred embodiment, specific antibodies may be employed in order to remove CXCL4, for instance from the circulation. This may for example be accomplished by plasmapheresis.

Antibodies against CXCL4 are also available in the art. They have been described for instance in Vandercapellen et al., J. Leukocyte Biol. 82, 1519 (2007) and are commercially available from R&D systems, 614 McKinley Place NE, Minneapolis MN 55413.

Given the teachings of the present disclosure, it will be within the routine skills of a person skilled in the art to design and develop materials and methods for removing or counteracting the effects of CXCL4 and/or DCs such that patients with autoimmune and/or autoinflammatory diseases such as rheumatoid arthritis, psoriatic arthritis, lupus erythematosus, systemic sclerosis, multiple sclerosis, etc may benefit from these materials and methods.

For that purpose, CXCL4 antagonists may advantageously employed. Antagonists for CXCL4 are known to the skilled person. As an example heparinase is disclosed and exemplified herein.

In another embodiment, the invention also relates to a compound capable of interfering with the in vivo production of CXCL4 for treatment or prevention of fibrosis in patients with autoimmune and/or autoinflammatory diseases such as rheumatoid arthritis, psoriatic arthritis, lupus erythematosus, systemic sclerosis, multiple sclerosis, etc. Compounds capable of interfering with the in vivo production of CXCL4 per se are known in the art. The invention therefore also relates to a method for treatment or prevention of fibrosis in patients with autoimmune and/or autoinflammatory diseases such as rheumatoid arthritis, psoriatic arthritis, lupus erythematosus, systemic sclerosis, multiple sclerosis, etc by interfering with the in vivo production of CXCL4. Such methods may encompass interference at the level of transcription or translation of CXCL4. Such methods are known in the art and may now advantageously be used in the treatment of autoimmune and/or autoinflammatory diseases such as rheumatoid arthritis, psoriatic arthritis, lupus erythematosus, systemic sclerosis, multiple sclerosis, etc.
A reduction in the level of CXCL4 in a patient's body may be accomplished by a therapy in vivo. Alternatively, patients with autoimmune and/or autoinflammatory diseases such as rheumatoid arthritis, psoriatic arthritis, lupus erythematosus, systemic sclerosis, multiple sclerosis, etc may also benefit from in vitro methods that reduce the level of CXCL4 in blood. Such may be done directly by applying a compound capable of binding to CXCL4 and contacting that compound with blood obtained from a patient with autoimmune and/or autoinflammatory diseases such as rheumatoid arthritis, psoriatic arthritis, lupus erythematosus, systemic sclerosis, multiple sclerosis, etc. It may also be accomplished by reducing the number of DC that produce the CXCL4 in patients with autoimmune and/or autoinflammatory diseases such as rheumatoid arthritis, psoriatic arthritis, lupus erythematosus, systemic sclerosis, multiple sclerosis, etc. This may also be accomplished in an in vitro method wherein the previously isolated blood or plasma from a patient with autoimmune and/or autoinflammatory diseases such as rheumatoid arthritis, psoriatic arthritis, lupus erythematosus, systemic sclerosis, multiple sclerosis, etc. is contacted with a compound or device capable of reducing or depleting the number of pDCs in solution. The invention therefore also relates to an in vitro method for reducing the level of CXCL4 and/or the number of pDCs in blood obtained from a patient with autoimmune and/or autoinflammatory diseases such as rheumatoid arthritis, psoriatic arthritis, lupus erythematosus, systemic sclerosis, multiple sclerosis, etc. In more detail, the invention relates to an in vitro method for reducing the level of CXCL4 in patients with autoimmune and/or autoinflammatory diseases such as rheumatoid arthritis, psoriatic arthritis, lupus erythematosus, systemic sclerosis, multiple sclerosis, etc comprising the steps of:
a. Providing said sample obtained from a patient with autoimmune and/or autoinflammatory diseases
b. Contacting the sample with a compound capable of binding to CXCL4
c. Allowing the compound to bind to CXCL4 in order to form a complex

Optionally, the complex may be removed from the sample, thereby reducing the level of CXCL4.

Devices capable of reducing the level of CXCL4 in blood are known in the art. Preferably, CXCL4 is removed from circulation, for instance using a commercially available technique provided by Miltenyi (Jongbloed et al., Arthritis Res Ther. 2006;8(1):R15).

Miltenyi produces and markets such a device which may advantageously be used in the new treatment according to the invention. Hence, the invention also relates to a device capable of reducing the level of CXCL4 in blood for the treatment or prevention of fibrosis in patients with scleroderma.

Alternatively, a method according to the invention may also comprise a step wherein the binding of CXCL4 to its receptor is blocked or otherwise prevented or down-regulated.or decreased. The invention therefore also provides a method for treatment or prevention of fibrosis in patients with autoimmune and/or autoinflammatory diseases such as scleroderma by interfering with the binding of CXCL4 to its receptor.

### Legend to the figure

**Figure 1****. CXCL4 augments TLR mediated responses.**
   DCs stimulated with CXCL4 for 6 days produce less inflammatory mediators (TNFa). However, when CXCL4 stimulated DCs are triggered by divers TLR ligands they produce markedly higher levels of TNFa to LPS compared to DCs who were not pre-stimulated.

## Claims

1. Compound capable of binding to CXCL4 for use in the treatment or prevention of fibrosis in patients with autoimmune and/or autoinflammatory diseases such as rheumatoid arthritis, psoriatic arthritis, lupus erythematosus, systemic sclerosis, multiple sclerosis, etc.

2. Compound for use according to claim 1 wherein the treatment includes the step of removing CXCL4.

3. Compound for use according to claims 1 or 2 wherein the compound is an antagonist of CXCL4.

4. Compound for use according to claims 1 - 3 wherein the compound is an antibody against CXCL4.

5. Compound for use according to claims 1 - 4 wherein the patients suffer from AID such as rheumatoid arthritis, psoriatic arthritis, lupus erythematosus, systemic sclerosis, multiple sclerosis, etc

6. In vitro method for reducing the level of CXCL4 from a patient with AID such as rheumatoid arthritis, psoriatic arthritis, lupus erythematosus, systemic sclerosis, multiple sclerosis, etc comprising the steps of
a. Providing said sample obtained from a patient with AID such as rheumatoid arthritis, psoriatic arthritis, lupus erythematosus, systemic sclerosis, multiple sclerosis, etc
b. Contacting the sample with a compound capable of binding to CXCL4
c. Allowing the compound to bind to CXCL4 in order to form a complex

7. In vitro method according to claim 6 additionally comprising the step of removing the complex from the sample, thereby reducing the level of CXCL4.

8. In vitro method according to claim 6 or 7 wherein the sample is blood, plasma or serum.

9. In vitro method according to claims 6 - 8 wherein the compound is an antibody against CXCL4.

10. Device capable of reducing the level of CXCL4 in a sample for use in the treatment or prevention of fibrosis in patients with AID such as rheumatoid arthritis, psoriatic arthritis, lupus erythematosus, systemic sclerosis, multiple sclerosis, etc.
